(19) Europäisches Patentamt / European Patent Office / Office européen des brevets

(11) **EP 4 635 530 A1**

(12) **EUROPEAN PATENT APPLICATION**

(43) Date of publication:
22.10.2025 Bulletin 2025/43

(21) Application number: 24170195.2

(22) Date of filing: 15.04.2024

(51) International Patent Classification (IPC):
*A61M 5/00* (2006.01)     *A61J 1/16* (2023.01)

(52) Cooperative Patent Classification (CPC):
A61M 5/008; A61J 1/16; A61M 5/002

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: SCHOTT Pharma Schweiz AG
9001 St. Gallen (CH)

(72) Inventors:
• **VASANTHAMOHAN, Nithan**
**9001 St. Gallen (CH)**
• **BERLINGER, Marcel**
**9001 St. Gallen (CH)**

(74) Representative: **Schott Corporate IP**
**Hattenbergstraße 10**
**55122 Mainz (DE)**

(54) **CARTRIDGE NEST, WHICH FEATURES INTEGRATED CENTERING OF THE CARTRIDGES**

(57) The present invention relates to a holding structure (100) for concurrently holding a plurality of primary packaging containers (200) for substances for pharmaceutical, medical or cosmetic applications, each primary packaging container (200) comprising an elongated cylindrical body region (201) having a longitudinal axis $L_{container}$ that runs in a longitudinal direction through the centre of the elongated cylindrical body region (201), the holding structure (100) comprising:
- a plurality of receptacles (101) for accommodating at least a section (201') of the elongated cylindrical body region (201) of the primary packaging container (200), each receptacle (101) having a depth **h**, an upper end (102) for inserting the primary packaging container (200) into the receptacle (101), a bottom end (103) having a holding portion (104) configured to limit an axial movement of the primary packaging container (200) in the receptacle (101), a circumferentially formed side wall (105) extending in a longitudinal direction from the upper end (102) to the bottom end (103) and a longitudinal axis $L_{receptable}$ that runs in a longitudinal direction through the centre of the receptacle (101);
- alignment elements (106) in each receptacle (101) that project radially into the receptacle (101) and that are adapted and arranged to contact a part of the outer surface of the elongated cylindrical body region (201) of a primary packaging container (200) accommodated in the receptacle (101);
wherein the alignment elements (106) in each receptacle (101) are adapted and arranged to contact a part of the outer surface of the elongated cylindrical body region (201) in such a way that a primary packaging container (200) accommodated in the receptacle (101) in a resting position is tilt by an angel α of not more than 2.4 degrees, the tilting angle α being the angle that is formed between longitudinal axis $L_{receptacle}$ and longitudinal axis $L_{container}$ of the primary packaging container (200) accommodated in the receptacle (101) and the resting position being the position in which the holding portion (104) limits the axial movement of the primary packaging container (200) accommodated in the receptacle (101).

The invention also relates to an arrangement comprising the holding structure (100) and a plurality of primary packaging containers (200), to a transport unit comprising the arrangement and a secondary packaging container and to the use of the holding structure (100).

**(Cont. next page)**

# Fig. 3

**Description**

The present invention relates to a holding structure for concurrently holding a plurality of containers, preferably a plurality of cartridges, for substances for pharmaceutical, medical or cosmetic applications, to an arrangement comprising the holding structure and a plurality of containers, to a transport unit comprising the arrangement and a secondary packaging container and to the use of the holding structure.

Containers made from glass and later on also from polymer have been applied for storing fluids and powders safely for a long time. In the last decades, the arts in which glass and polymer containers are used for transporting fluids and powders have become increasingly diverse and sophisticated. One such art is the technical field of the present application: pharmaceutical packaging. In the pharmaceutical industry, containers - such as vials, syringes, ampoules, and cartridges - are used as primary packaging for all kinds of pharmaceutically relevant compositions, in particular drugs, such as parenteralia including vaccines, and also for cosmetic compositions, in particular cosmetical compositions which are to be injected into the skin.

In the processing of containers for use in pharmaceutical or cosmetical applications, generally so-called nested solutions are often preferred nowadays, where a holding structure for containers (also referred to as nest) is used for concurrently holding or supporting a plurality of primary packaging containers in a given configuration. An example of a known nested solution is commercially available from Schott Pharma AG under the tradename SCHOTT iQ® platform. The nest with the primary packaging containers is usually delivered to a customer, such as a pharmaceutical company or filler, packaged in a transport or packaging container (also referred to as tub). For further processing the primary packaging containers, the tub is opened. Further processing the primary packaging containers often includes automated steps of removing the primary packaging containers from the tub; filling the primary packaging containers with a composition, e.g. a pharmaceutical or cosmetical composition; and closing the pre-filled primary packaging containers.

For filling the containers accommodated in the holding structures filling nozzles are used that are, to a certain extent, inserted into the opening of the containers. In such an automated filling process the containers must be positioned as parallel to the axis of the filling nozzles as possible. If this is not the case and the container is positioned at a slight angle, fill level fluctuations or even damage to the filling nozzle can occur if the filling needle collides with the inclined container. Also, an incorrect positioning of the containers can also lead to failures or errors when lifting the container before they are filled as the crimp can get stuck on the holding feature.

In automated filling processes of the prior art a separate positioning aid in the form of a perforated plate is used that guaranties a parallel positioning of each container accommodated in the supporting structure. Such a position aid, however, not only represents an additional component that is needed in the automated filling process but may also be problematic as the introduction of the open ends of the containers into the perforated plate is made more difficult if the containers are tilt to a certain extend in the holding structure. Also, handling of machine components such as perforated plates above the open containers which may already comprise the medicament can also lead to contamination within the filled container. Finally, depending on the material quality of the perforated plate that is commonly used to ensure a proper alignment of the containers, friction between the perforated plate and the container may cause abrasion on the glass surface and thus damage of the container.

In general, it is an object of the present invention to at least partly overcome a disadvantage arising from the prior art.

It is particularly an object of the present invention to provide a nested solution of pharmaceutical, medical, or cosmetic containers which allows filling of the containers accommodated in the nest as efficiently as possible without the necessity of using separate positioning means. More particularly, the nested solution should allow filling of the containers accommodated in the nest with no damage of the containers by the needle used to fill the container and with as little fill level fluctuations as possible, even if no additional separate positioning means are used. Also, the nested solution should allow the packaging containers to be inserted into and removed from the nest with as little abrasion of the outer surface of the packaging containers as possible.

A contribution to at least partly solving at least one, preferably more than one, of the above objects is made by the independent claims. The dependent claims provide preferred embodiments which contribute to at least partly solving at least one of the objects.

|1a| A contribution to solving at least one of the objects according to the invention is made by a 1st embodiment of holding structure for concurrently holding a plurality of primary packaging containers for substances for pharmaceutical, medical or cosmetic applications, each primary packaging container comprising an elongated cylindrical body region having a longitudinal axis $L_{container}$ that runs in a longitudinal direction through the centre of the elongated cylindrical body region, the holding structure comprising:

- a plurality of receptacles for accommodating at least a section of the elongated cylindrical body region of the primary packaging container, each receptacle having a depth **h,** an upper end for inserting the primary packaging container into the receptacle, a bottom end having a holding portion configured to limit an axial movement of the primary packaging container in the receptacle, a circumferentially formed side wall extending in a longitudinal direction from the upper end to the bottom end and a longitudinal axis $L_{receptable}$ that runs in a longitudinal direction through the centre of the receptacle;

- alignment elements in each receptacle that project radially into the receptacle and that are adapted and arranged to contact a part of the outer surface of the elongated cylindrical body region of a primary packaging container accommodated in the receptacle;

wherein the alignment elements in each receptacle are adapted and arranged to contact a part of the outer surface of the elongated cylindrical body region in such a way that a primary packaging container accommodated in the receptacle in a resting position is tilt by an angel $\alpha$ of not more than 2.4 degrees, preferably not more than 2.2 degrees, more preferably not more than 2.0 degrees, even more preferably not more than 1.8 degrees, even more preferably not more than 1.6 degrees, even more preferably not more than 1.4 degrees, even more preferably not more than 1.2 degrees, and most preferably not more than 1 degree, the tilting angle $\alpha$ being the angle that is formed between longitudinal axis $L_{receptacle}$ and longitudinal axis $L_{container}$ of the primary packaging container accommodated in the receptacle and the resting position being the position in which the holding portion limits the axial movement of the primary packaging container accommodated in the in the receptacle. Most preferably, a primary packaging container accommodated in the receptacle in a resting position is not tilt at all, *i.e.,* $\alpha$ is 0 degrees.

The term *"a plurality of receptacles for receiving the primary packaging containers therein at least in sections"* as used herein indicates that the depth **h** of the receptacle is smaller than the hight of the primary packaging container to be accommodated in the receptacle so that in the resting position portions of the primary packing container protrude from the receptacle.

To ensure that the primary packaging containers in the holding structure are tilt as little as possible in the receptacles the skilled person would have considered increasing the guide length and therefore the depth of the receptacles or reducing the internal diameter of the receptacles in which the primary packaging containers are accommodated. However, these measures would lead to particle generation in the manufacturing process, *i.e.* in the injection moulding process, due to abrasion during the demoulding process. In addition, inserting and removing the primary packaging containers into and from the holding structure with a large contact surface and low pocket internal diameter can lead to various defects, such as the generation of particles, the destruction of the primary packaging container surface or a jamming of the primary packaging containers in the holding structure.

Surprisingly, however, it has been observed that an as vertical possible alignment of the primary packaging containers in the holding structure can be ensured by the provision of alignment elements that are arranged in the receptacles and that contact only a part of the outer surface of the primary packaging containers, more precisely only a part of the outer surface of the elongated cylindrical body region of these containers. Thus, to achieve the parallel alignment of the primary packaging containers in the holding structure it is neither necessary to increase the depth of the receptacles nor to decrease the diameter of the receptacles or to use separate positioning means (although such positioning means may nevertheless be used as the alignment elements also facilitate the introduction of the containers in the holes of the positioning means).

|2a| According to a preferred embodiment of the holding structure according to the present invention, the alignment elements in each receptacle are adapted and arranged to contact a part of the outer surface of the elongated cylindrical body region in such a way that a primary packaging container accommodated in the receptacle in a lifting position is still tilt by an angel $\alpha'$ of not more than 2.4 degrees, preferably not more than 2.2 degrees, more preferably not more than 2.0 degrees, even more preferably not more than 1.8 degrees, even more preferably not more than 1.6 degrees, even more preferably not more than 1.4 degrees, even more preferably not more than 1.2 degrees and most preferably not more than 1.0 degree, the tilting angle $\alpha'$ being the angle that is formed between longitudinal axis $L_{receptacle}$ and longitudinal axis $L_{container}$ of the primary packaging container accommodated in the receptacle and the lifting position being the position in which the primary packaging container is lifted out of the resting position in a direction away from the bottom end to a lifting height **a,** wherein **a** is 10 to 50 %, preferably 20 to 48 % and more preferably 25 to 45 % of the depth **h** of the receptacle. Most preferably, also in the lifting position a primary packaging container accommodated in the receptacle is not tilt at all, *i.e.,* $\alpha'$ is 0 degrees. Preferably, **a** is in the range from 13 to 20 mm. This embodiment is a 2nd embodiment of the holding structure according to the present invention, that preferably depends on the 1st embodiment.

|3a| According to a further preferred embodiment of the holding structure according to the present invention, holding protrusions acting as holding portions are provided at the bottom ends of the receptacles, which extend radially inwards into the receptacles. Each receptacle may, for example, have two holding protrusions which are diametrically opposite to each other (as shown in Figures 3 and 4). In principle, a single holding protrusion is also sufficient for limiting an axial movement of the primary packaging containers in the receptacles, which may also be designed to be circumferential or essentially circumferential. This embodiment is a 3rd embodiment of the holding structure according to the present invention, that preferably depends on the 1st or the 2nd embodiment.

|4a| According to a further preferred embodiment of the holding structure according to the present invention, the holding structure is formed by injection moulding from a plastic material, wherein the side walls of the receptacles are inclined at a draft angle $\gamma$ in relation to longitudinal axis $L_{receptable}$ so that the inner diameter $d$ of the receptacle constantly increases from the bottom end to the upper end. In this context the draft angle $\gamma$ is preferably in the range from 0.01 to 1 degree, more preferably in the range from 0.025 to 0.75 degrees, even more preferably in the range from 0.05 to 0.5 degrees and most preferably in the range 0.075 to 0.3 degrees. Generally, the draft angle $\gamma$ is of the order of one degree or less to facilitate the demoulding of the holding structure from an injection mould during manufacture by injection moulding. According to a particular embodiment of the the holding structure according to the present invention, the side walls of the receptacles are not inclined at all (draft angle $\gamma$ = 0 degree) in at the bottom end of the receptacles, preferably up to a height $h_x$ in the range from 1 to 5 mm, preferably 2 to 4 mm measured from the bottom end of the receptacles and wherein beginning at the height $h_x$ the side walls of the receptacles are inclined by the above-mentioned draft angles up to the upper and of the receptacles. Thus, in such an embodiment the inner diameter $d$ of the receptacle is constant in the area from the bottom of the receptacle up to height $h_x$ and constantly increases from the height $h_x$ to the upper end. This embodiment is a 4th embodiment of the holding structure according to the present invention, that preferably depends on any of the 1st to the 3rd embodiment.

|5a| According to a further preferred embodiment of the holding structure according to the present invention, the holding structure is formed by injection moulding from a plastic material, wherein the holding structure is designed in such a manner that a two-part original mould with a lower mould and an upper mould is used for production by injection moulding from the plastic material such that the side walls of the receptacles are not inclined at a draft angle $\gamma$ in relation to longitudinal axis $L_{receptable}$ such that the inner diameter $d$ of the receptacle constantly increases from the bottom end to the upper end. In this embodiment the inner diameter $d_{bottom}$ of the receptacle at the bottom end either corresponds to the inner diameter $d_{top}$ at the upper end or is even larger than the inner diameter $d_{top}$ at the upper end. This embodiment is a 5th embodiment of the holding structure according to the present invention, that preferably depends on any of the 1st to the 3rd embodiment.

|6a| According to a further preferred embodiment of the holding structure according to the present invention,

- the receptacles are arranged in a regular arrangement,

- an upper side of the holding structure is formed as a plate-shaped support, and

- the receptacles project vertically from the plate-shaped support;

wherein the side wall is formed as a common partition between each two directly adjacent receptacles of the plurality of receptacles. This embodiment is a 6th embodiment of the holding structure according to the present invention, that preferably depends on any of the 1st to the 5th embodiment.

|7a| According to a further preferred embodiment of the holding structure according to the present invention the partition walls, viewed in a cross-section, are each formed in one piece and without openings. This embodiment is a 7th embodiment of the holding structure according to the present invention, that preferably depends on the 6th embodiment.

|8a| According to a further preferred embodiment of the holding structure according to the present invention, the partition walls, viewed in a cross-section, are formed in two or more separate pieces, wherein the partition wall comprises openings between two neighboured pieces of the partition wall. This embodiment is an 8th embodiment of the holding structure according to the present invention, that preferably depends on the 6th embodiment.

|9a| According to a further preferred embodiment of the holding structure according to the present invention, the receptacles are polygonal or circular in shape when viewed from above. The receptacles may, for example, be hexagonal in shape when viewed in a plan view and arranged directly adjacent to each other in a regular arrangement with hexagonal symmetry. The receptacles may also be octagonal in shape when viewed in a plan view, with four adjacent receptacles arranged in a rhombic arrangement. A particular preferred shape of the receptacles is a rhombic shape. This embodiment is a 9th embodiment of the holding structure according to the present invention, that preferably depends on any of the 1st to the 8th embodiment.

|10a| According to a further preferred embodiment of the holding structure according to the present invention,

- **A** is the surface of the section of the elongated cylindrical body region of the primary packaging container that is accommodated in the receptacle of the holding structure;

- **B** is the part of surface **A** that can come into contact with a surface of the alignment elements in a given receptacle if the primary packaging container is accommodated in the receptacle in the resting position;

wherein **B/A** is less than 0.1, preferably less than 0.05, more preferably less than 0.03, even more preferably less than 0.02 and most preferably less than 0.01. This embodiment is a 10th embodiment of the holding structure according to the present invention, that preferably depends on any of the 1st to the 9th embodiment.

|11a| According to a further preferred embodiment of the holding structure according to the present invention, in each receptacle the alignment elements comprise a plurality of longitudinal alignment elements running in a direction from the upper end to the bottom end of the receptacle and extending over at least 50 %, preferably at least 75 % and more preferably at least 90 % of the entire depth **h** of the receptacle, wherein the alignment elements are adapted and arranged to contact a part of the outer surface of the primary packaging container over at last a certain part of the length of the elongated cylindrical body region that is accommodated in the receptacle. The longitudinal alignment elements running in a direction from the upper end to the bottom end of the receptacle can be designed as continuous alignment elements which are not interrupted on their way from the upper to the lower end. However, they can also consist of several longitudinal elements arranged one behind the other and separated from each other by interruptions Also, in each receptacle there are preferably two or more of these longitudinal alignment elements, more preferably 4 to 6 of these longitudinal alignment elements, that are equally distributed at angular spacings from one another. This embodiment is an 11th embodiment of the holding structure according to the present invention, that preferably depends on any of the 1st to the 10th embodiment.

|12a| According to a further preferred embodiment of the holding structure according to the present invention, in each receptacle there are one more positions $P_x$ at which at least two alignment elements are provided with a concave recess the curvature of which corresponds to the outer diameter of the primary packaging container to be accommodated in the receptacle, wherein the alignment elements are adapted and arranged to contact the outer surface of the primary packaging container over at least a part of the circumference of the elongated cylindrical body region that is accommodated in the receptacle. In case of two or more positions $P_x$ at which these alignment elements are provided, these positions differ in the height at which they are arranged along longitudinal axis $L_{receptable}$ of the receptacle. Preferably, at least one of these positions $P_x$ is located in the upper third, even more preferably in the upper quarter of the receptacle. This embodiment is a 12th embodiment of the holding structure according to the present invention, that preferably depends on any of the 1st to the 10th embodiment.

|13a| According to a further preferred embodiment of the holding structure according to the present invention, in each receptacle there are one more positions $P_x$ at which at least two alignment elements are provided, wherein the alignment elements are adapted and arranged to punctually contact the outer surface of the primary packaging container on at least a part of the elongated cylindrical body region that is accommodated in the receptacle. In case of two or more positions $P_x$ at which these alignment elements are provided, these positions again differ in the height at which they are arranged along longitudinal axis $L_{receptacle}$ of the receptacle. In this context it is also preferred that at a given position $P_x$ there is a plurality of punctual alignment elements that are equally distributed at angular spacings from one another. More preferably, the punctual alignment elements are located at least at a first position $P_1$ in the area of the upper end of the receptacle, preferably in the upper third, more preferably in the upper quarter of the receptacle, and at a second position $P_2$ in the area of the bottom end of the receptacle, preferably in the lower third, more preferably in the lower quarter of the receptacle. In case of punctual alignment elements that are located at two positions $P_1$ (in the area of the upper end) and $P_2$ (in the area of the bottom end) it is also preferred that the alignment elements at position $P_1$ are located along a first circle located in a first plane and the alignment elements at position $P_2$ are located along a second circle located in a second plane, the first and the second plane being parallel to each other and being perpendicular to longitudinal axis $L_{receptacle}$, wherein the diameter $\varnothing_1$ of the first circle at position $P_1$ is larger than the diameter $\varnothing_2$ of the second circle at position $P_2$. In this context it is preferred that the term

$$\Delta h \, / \, |\varnothing_2 - \varnothing_1|$$

is in the range from 0 to 0.01, more preferably in the range from 0 to 0.007 and most preferably in the range from 0 to 0.0035, $\Delta h$ being the distance between position $P_1$ and position $P_2$ in a direction parallel to longitudinal axis $L_{receptacle}$. This embodiment is a 13th embodiment of the holding structure according to the present invention, that preferably depends on any of the 1st to the 10th embodiment.

|14a| According to a further preferred embodiment of the holding structure according to the present invention, the alignment elements are located on the top of the side walls. This embodiment is a 14th embodiment of the holding structure according to the present invention, that preferably depends on any of the 1st to the 13th embodiment.

|15a| According to a further preferred embodiment of the holding structure according to the present invention, the holding structure further comprises

- guide portions adapted and arranged to guide the primary packaging containers as they are inserted into the receptacles, the guide portions being formed as guiding ribs extending in a longitudinal direction of the receptacles,

wherein the alignment elements are located on the top of the guide portions. In this context it may also be advantageous if lead-in bevels are formed at upper ends of the guide portions which are inclined relative to the guide portions as disclosed in US 11,464,702 B2, wherein the alignment elements may also be located on the top of these lead-in bevels. This embodiment is a 15th embodiment of the holding structure according to the present invention, that preferably depends on any of the 1st to the 13th embodiment.

|1b| A contribution to solving at least one of the objects according to the invention is made by a 1st embodiment of an arrangement comprising:

- the holding structure according to the present invention, preferably the holding structure according to any of the 1st to the 15th embodiment;

- a plurality of primary packaging containers, each primary packaging container being accommodated in one of the receptacles of the holding structure.

Preferably, each of the primary packaging containers is, preferably non-destructively, detachably accommodated in one of the receptacles.

|2b| According to a preferred embodiment of the arrangement according to the present invention, the plurality of primary packaging containers comprises a number of primary packaging containers which is in the range from 4 to 500, preferably from 9 to 400, more preferably from 12 to 300, more preferably from 16 to 200, more preferably from 16 to 160, more preferably from 16 to 100, more preferably from 16 to 90, more preferably from 16 to 80, more preferably from 16 to 70, even more preferably from 16 to 60, most preferably from 20 to 160. This embodiment is a 2nd embodiment of the arrangement according to the present invention, that preferably depends on the 1st embodiment.

|3b| According to a further preferred embodiment of the arrangement according to the present invention, each of the primary packaging containers contains a pharmaceutical, medical or cosmetic composition. This embodiment is a 3rd embodiment of the arrangement according to the present invention, that preferably depends on the 1st or the 2nd embodiment.

|4b| According to a further preferred embodiment of the arrangement according to the present invention, each of the primary packaging containers is closed. This embodiment is a 4th embodiment of the arrangement according to the present invention, that preferably depends on any of the 1st to the 3rd embodiment.

|5b| According to a further preferred embodiment of the arrangement according to the present invention, each of the primary packaging containers comprises, in the following sequence along its length,

　　a) a first end part, comprising a discharge opening,

　　b) an elongated cylindrical body region, and

　　c) a further end part.

This embodiment is a 5th embodiment of the arrangement according to the present invention, that preferably depends on any of the 1st to the 4th embodiment.

|6b| According to a further preferred embodiment of the arrangement according to the present invention, the further end part is a standing base, or comprises a further opening, or both. A preferred further orifice is designed to accommodate a plunger. This embodiment is a 6th embodiment of the arrangement according to the present invention, that preferably depends on the 5th embodiment.

|7b| According to a further preferred embodiment of the arrangement according to the present invention, for each of the primary packaging containers, an area of the further opening is more than an area of the discharge opening. This embodiment is an 7th embodiment of the arrangement according to the present invention, that preferably depends on the 6th embodiment.

|8b| According to a further preferred embodiment of the arrangement according to the present invention, the further end part further comprises a rim which projects laterally from the elongated cylindrical body region and at least partially, preferably fully, hems the further opening. This embodiment is a 8th embodiment of the arrangement according to the present invention, that preferably depends on the 6th or the 7th embodiment.

|9b| According to a further preferred embodiment of the arrangement according to the present invention, the first end part of each of the primary packaging containers comprises a connecting element, wherein the connecting element comprises a thread for connecting an auxiliary part to the primary packaging container. A preferred auxiliary part is one selected from the group consisting of a needle, a nozzle, and a tubing, or a combination of at least two thereof. A preferred needle is a hypodermic needle. This embodiment is a 9th embodiment of the arrangement according to the present invention, that preferably depends on any of the 5th to the 8th embodiment.

|10b| According to a further preferred embodiment of the arrangement according to the present invention, the connecting element comprises a thread for connecting an auxiliary part to the respective primary packaging container. This preferred embodiment is an 10th embodiment of the invention, that preferably depends on the 9th embodiment of the invention.

|11b| According to a further preferred embodiment of the arrangement according to the present invention, the first end part of each of the primary packaging containers comprises a male part of a taper fitting. A preferred taper fitting is a Luer taper. Generally, the Luer taper may comprise a thread or not. This embodiment is an 11th embodiment of the arrangement according to the present invention, that preferably depends on any of the 5th to the 10th embodiment.

|12b| According to a further preferred embodiment of the arrangement according to the present invention, the male part of the taper fitting comprises a thread.

Preferably, the thread is arranged in a sleeve. This preferred embodiment is a 12th embodiment of the invention, that preferably depends on the 11th embodiment of the invention.

|13b| According to a further preferred embodiment of the arrangement according to the present invention, for each of the primary packaging containers, throughout the elongated cylindrical body region a thickness of a container wall is in a range from ± 0.3 mm, preferably ± 0.2 mm, more preferably ± 0.15 mm, more preferably ±0.1 mm, most preferably ± 0.08 mm, in each case based on a mean value of the thickness of the container wall in the body part of the respective primary packaging container. This embodiment is a 13th embodiment of the arrangement according to the present invention, that preferably depends on any of the 5th to the 12th embodiment.

|14b| According to a further preferred embodiment of the arrangement according to the present invention, for each of the primary packaging containers, throughout the elongated cylindrical body region a thickness of a container wall is in a range from 0.2 to 3 mm, preferably from 0.3 to 2.5 mm, more preferably from 0.4 to 2.2 mm. This embodiment is a 14th embodiment of the arrangement according to the present invention, that preferably depends on any of the 5th to the 13th embodiment.

|15b| According to a further preferred embodiment of the arrangement according to the present invention, throughout the elongated cylindrical body region a thickness of a container wall is in a range from 1.0 to 1.1 mm. In a further preferred embodiment, for each of the primary packaging containers, throughout the elongated cylindrical body region a thickness of a container wall is in a range from 1.4 to 1.8 mm. In yet a further preferred embodiment, for each of the primary packaging containers, throughout the elongated cylindrical body region a thickness of a container wall is in a range from 0.6 to 2.0 mm. This embodiment is a 15th embodiment of the arrangement according to the present invention, that preferably depends on any of the 5th to the 14th embodiment.

|16b| According to a further preferred embodiment of the arrangement according to the present invention, each of the primary packaging containers has a container interior with a volume in a range from 0.5 to 100 ml, preferably from 0.8 to 100 ml, more preferably from 1 to 50 ml, even more preferably from 1 to 10 ml. This embodiment is a 16th embodiment of the arrangement according to the present invention, that preferably depends on any of the 1st to the 15th embodiment.

|17b| According to a further preferred embodiment of the arrangement according to the present invention, the primary packaging containers are selected from the group consisting of vials, syringes, cartridges, and ampoules, or a combination of at least two thereof. A preferred cartridge is designed for being used as a reservoir in a, preferably portable, medical device. A preferred portable medical device is an insulin pump. This embodiment is an 17th embodiment of the arrangement according to the present invention, that preferably depends on any of the 1st to the 16th embodiment.

|18b| According to a further preferred embodiment of the arrangement according to the present invention, each of the primary packaging containers comprises a container wall which at least partially surrounds a container interior, wherein the container wall comprises, preferably consists of, a glass, or a polymer, or both. This embodiment is an 18th embodiment of the arrangement according to the present invention, that preferably depends on any of the 1st to the 17th embodiment.

|19b| According to a further preferred embodiment of the arrangement according to the present invention, the polymer is a cyclic olefin copolymer, or a cycloolefin polymer, or a mixture thereof. This embodiment is a 19th embodiment of the arrangement according to the present invention, that preferably depends on the 18th embodiment.

|20b| According to a further preferred embodiment of the arrangement according to the present invention, the glass is of a type selected from the group consisting of a borosilicate glass, preferably a type I glass; an aluminosilicate glass; and fused silica; or of a combination of at least two thereof. This embodiment is a 20th embodiment of the arrangement according to the present invention, that preferably depends on the 18th embodiment.

|21b| According to a further preferred embodiment of the arrangement according to the present invention, the primary packaging containers have been decontaminated, preferably sterilized. Preferably, the arrangement has been decontaminated, preferably sterilized. In the context of the present application, decontamination is defined as an umbrella term for reducing the amount of microbes and biological agents, such as fungi, bacteria, viruses, spore forms, prions, unicellular eukaryotic organisms, etc. The special terms disinfection and sterilization differ in the amount of reduction of these. While disinfection only reduces the amount of said contaminants, sterilization effectively kills, deactivates, or eliminates all forms of life and other biological agents which are present, i.e., a reduction of 100 %. Hence, disinfection is less effective than sterilization. This embodiment is a 21st embodiment of the arrangement according to the present invention, that preferably depends on any of the 1st to the 20th embodiment.

|1c| A contribution to solving at least one of the objects according to the invention is made by a 1st embodiment of a transport unit comprising:

- the arrangement according to the present invention, preferably the arrangement according to any of its 1st to the 21st embodiment, and

- a secondary packaging container,

wherein the holding structure and the plurality of primary packaging containers are arranged completely in the secondary packaging container.

**|2c|** According to a preferred embodiment of the transport unit according to the present invention, a container body of the secondary packaging container comprises

- a container opening, and

- a container base which is opposite to the container opening in the longitudinal direction.

This embodiment is a 2nd embodiment of the transport unit according to the present invention, that preferably depends on the 1st embodiment.

**|3c|** According to a further preferred embodiment of the transport unit according to the present invention, the first end parts, or the further end parts of the primary packaging containers face the container opening. This embodiment is a 3rd embodiment of the transport unit according to the present invention, that preferably depends on the 2nd embodiment.

**|4c|** According to a further preferred embodiment of the transport unit according to the present invention, the secondary packaging container is a tub or tub-shaped. This embodiment is a 4th embodiment of the transport unit according to the present invention, that preferably depends on any of the 1st to the 3 rd embodiment.

**|5c|** According to a further preferred embodiment of the transport unit according to the present invention, the secondary packaging container is closed by a lid. Preferably, the lid is joined to the secondary packaging container. A preferred lid is a multi-layer sheet. Additionally, or alternatively preferred, the lid is gas-permeable. This embodiment is a 5th embodiment of the transport unit according to the present invention, that preferably depends on any of the 1st to the 4th embodiment.

**|6c|** According to a further preferred embodiment of the transport unit according to the present invention, the transport unit further comprises a, preferably closed, outer packaging, wherein the secondary packaging container is arranged in the outer packaging. A preferred outer packaging is a pouch, preferably made from a plastic film. Additionally, or alternatively preferred, the outer packaging provides a barrier against a permeation of an inert gas. Additionally, or alternatively preferred, the outer packaging is hermetically sealed. Additionally, or alternatively preferred, the outer packaging is less permeable for the inert gas than the lid. Particularly preferred the outer packing provides a barrier action against a permeation of the inert gas, whereas the lid is permeable for the inert gas. This a 6th embodiment of the transport unit according to the present invention, that preferably depends on any of the 1st to the 5th embodiment.

**|7c|** According to a further preferred embodiment of the transport unit according to the present invention, the outer packaging comprises an atmosphere which comprises an inert gas at a proportion of at least 50 vol.-%, preferably at least 60 vol.-%, preferably at least 70 vol.-%, more preferably at least 80 vol.-%, even more preferably at least 90 vol.-%, most preferably at least 95 vol.-%, in each case based on a volume of the atmosphere. This preferred embodiment is a 7th embodiment of the transport unit according to the present invention, that preferably depends on the 6th embodiment.

**|1d|** A contribution to solving at least one of the objects according to the invention is made by a use of the holding structure of the invention, preferably of a holding structure according to any of the 1st to 15th embodiment, or of the arrangement of the invention, preferably of the arrangement according to any of the 1st to the 21st embodiment, or of the transport unit of the invention, preferably of the transport unit according to any of the 1st to the 7th embodiment, in each case for storing or transporting the plurality of primary packaging containers. In a preferred embodiment of the use, each of the primary packaging containers contains a pharmaceutical, medical or cosmetic composition.

**|1e|** A contribution to solving at least one of the objects according to the invention is made by a process for filling primary packaging containers with a pharmaceutical, medical, or cosmetic composition, the process comprising the steps:

A) providing an arrangement according to the present invention, preferably an arrangement according to any of the 1st to the 21st embodiment,

B) filling each primary packaging container accommodated in the receptacles of the holding structure with the liquid pharmaceutical composition in an automated filling machine.

**|2e|** According to a preferred embodiment of the process for filling primary packaging containers, in the automated filling machine a needle is positioned above the opening of the primary packaging container, followed by an injection of the pharmaceutical, medical, or cosmetic composition through the needle into the container, wherein the primary packaging container is filled with the pharmaceutical, medical, or cosmetic composition following the positioning of the needle above the corresponding primary packaging container. This a 2nd embodiment of the process for filling primary packaging containers according to the present invention, that preferably depends on the 1st embodiment.

**|1f|** A contribution to solving at least one of the objects according to the invention is made by a use of the holding structure of the invention, preferably of a holding structure according to any of the 1st to 15th embodiment, for holding the plurality of primary packaging containers in a step of filling the primary packaging containers with a pharmaceutical, medical, or cosmetic composition.

## Holding Structure

The holding structure of the invention may, generally, be any device which, for the skilled person, comes into consideration for holding the plurality of primary packaging containers. A preferred holding structure is a carrier structure of a so-called nested solution as they are generally known in the technical field of transport packaging for medical, pharmaceutical and cosmetical primary packaging containers. An example of a known nested solution is commercially available from Schott Pharma AG under the tradename SCHOTT iQ® platform. A preferred holding structure has been prepared by deep drawing or injection moulding, where injection moulding is particularly preferred. Additionally, or alternatively preferred, the holding structure is made from one or more plastics. Preferably, the plate-shaped carrier element is in one piece with the plurality of receptacles. Further preferably, the holding device is of a one-piece design. Preferably, the receptacles form a regular pattern in a top view on the holding device.

## Alignment Element

The alignment elements serve to prevent, or at least minimise, tilting of the primary packaging containers held in the receptacles of the holding structure. Tilting of the primary packaging containers occurs when an angle other than zero is included between the longitudinal axis $L_{container}$ of the primary packaging container and the longitudinal axis $L_{receptacle}$ of the receptacle that accommodates the primary packaging container (= tilting angle a). The longitudinal axis $L_{con\text{-}tainer}$ of the primary packaging container is the axis that runs through the centre of the elongated cylindrical body region of the primary packaging container, which is preferably rotationally symmetrical about this axis, while the longitudinal axis $L_{receptacle}$ of the receptacle is the axis that runs centrally and perpendicularly through the receptacle in the elongated direction along the depth **h** of the receptacle.

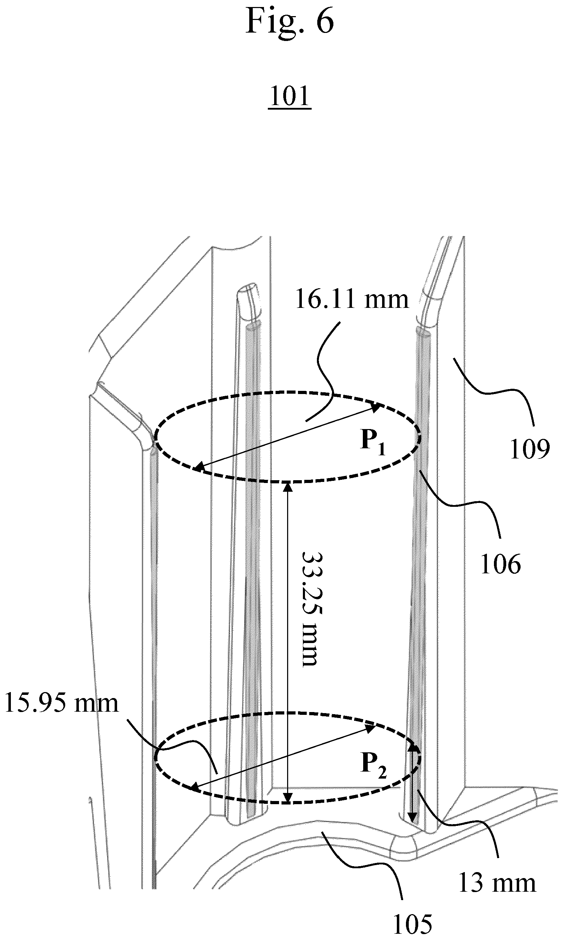

The alignment elements can have any shape that appears suitable to the person skilled in the art in order to prevent the containers from tilting in the receptacle. For example, they may extend in an elongate direction from the upper to the lower end of the receptacle, with two or more such elongate alignment elements being evenly spaced from one another around the circumference of the receptacle (as shown in Figure 6). In such an embodiment it is particularly advantageous to apply the alignment elements to the guide elements arranged in a similar manner. Preferably, the internal diameter of the receptacles, which is ultimately defined by the alignment elements, is smaller in the upper area of the receptacles than in the lower area.

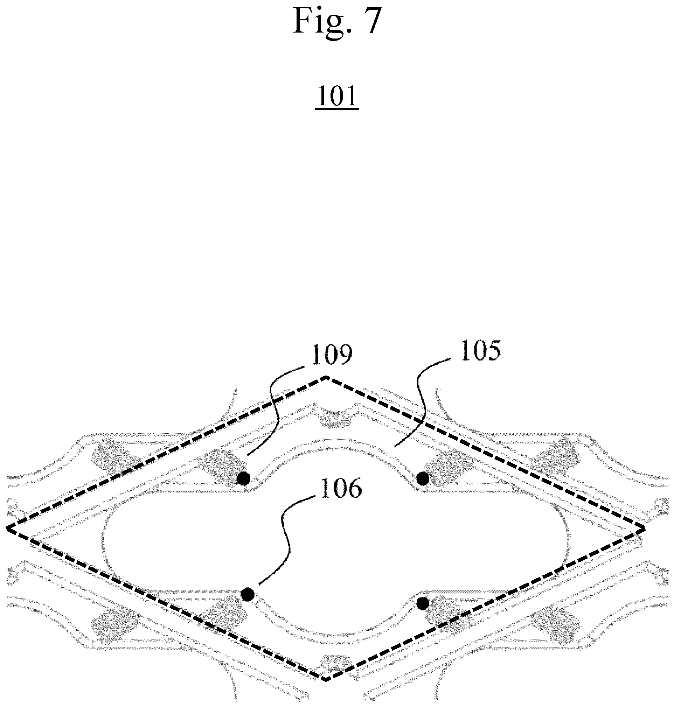

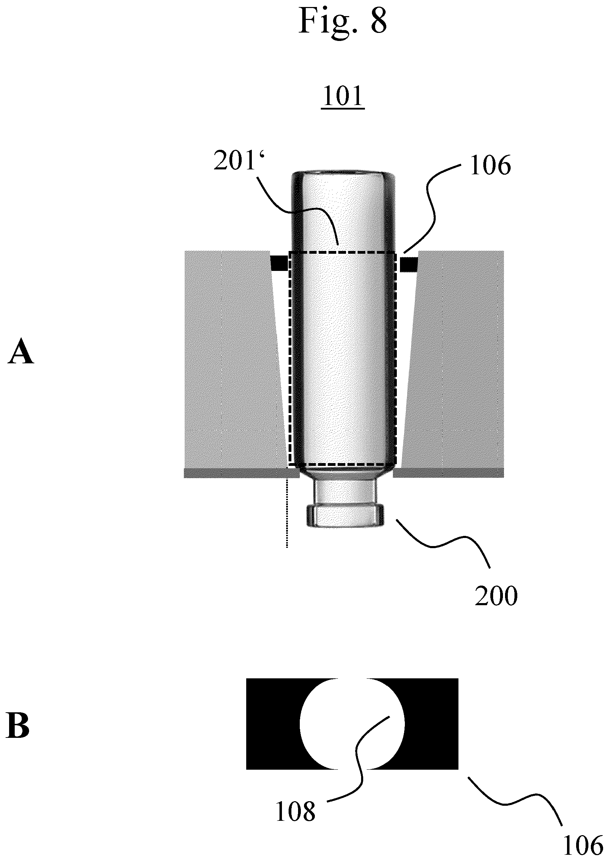

According to a further embodiment, the alignment elements can also have concave recesses whose curvature is adapted to the outer radius of the primary packaging containers, so that these alignment elements contact the primary packaging containers at certain positions along at least a part of their circumference (as shown in Figure 7). Such alignment elements may be arranged locally at one or more positions along the elongate axis of the receptacles, but preferably at least in the upper region of the receptacles, particularly preferably in the upper third, even more preferably in the upper quarter, wherein at a given position $P_x$ along the height of the receptacle preferably two such alignment elements arranged opposite one another are provided (as also shown in Figure 8).

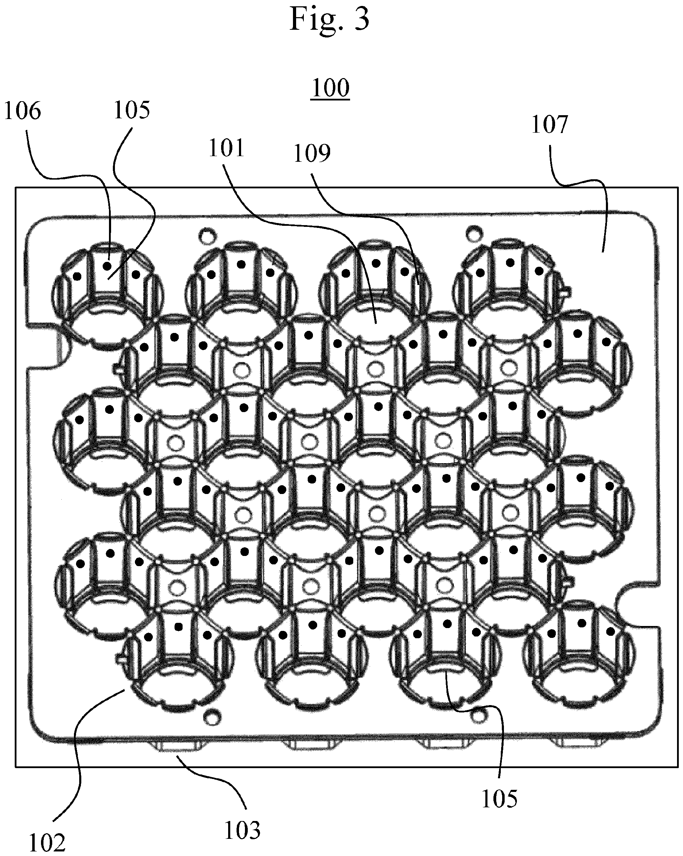

Particularly advantageous, however, are embodiments in which the alignment elements merely enable a point-like contact with the outer surface of the primary packaging containers, such alignment elements also being arranged locally at one or more positions $P_x$ along the height of the receptacle, preferably at least in the upper region of the receptacle, particularly preferably in the upper third, even more preferably in the upper quarter, wherein at a given position $P_x$ two, three, four, five, six or more such alignment elements arranged in a circular manner and with uniform spacing between each other (as shown in Figure 3). If the contact is only point-shaped, the friction between the alignment elements and the outer wall of the primary packaging container during insertion and removal of the container can be minimised. In principle, the size of the contact surface between the alignment elements and the primary packaging container, or more precisely between the alignment elements and the elongated cylindrical body region of the primary packaging container, should be as small as possible.

The alignment elements are preferably made of the same material as the holding structure itself. The alignment elements can also already be applied in an injection moulding process or in a 3D-printing process used to produce the holding structure themselves, or they can be applied subsequently by 3D-printing, bonding, or welding to corresponding surface areas in the receptacles of the holding structure produced by 3D-printing or injection, moulding or deep-drawing.

## Plate-Shaped Carrier Element

The term "plate-shaped" refers to a width and a length of the carrier element each being at least 5 times, preferably at least 10 times, more preferably at least 50 times, most preferably at least 100 times, more than a thickness of the carrier element. Preferably, the thickness of the carrier element is in the range from 0.5 to 5 mm, more preferably from 0.5 to 3 mm, even more preferably from 0.5 to 2 mm, most preferably from 0.8 to 2 mm. Additionally or alternatively preferred, the carrier element has an essentially flat top surface or an essentially flat bottom surface or both.

Here, the term "plate-shaped carrier element" refers to an essentially flat element without any optional macroscopic protrusions on the top or bottom surface of the carrier element. Preferably, the top and bottom surfaces of the plate-shaped carrier element are essentially rectangular. Here, "essentially" means that the top or bottom surfaces may gave rounded corners and/or recesses at a rim area.

Side Wall of the Receptacle

The receptacle wall of a receptacle, preferably, consists of all wall elements of the receptacle. Here, the receptacle wall may enclose the receptacle interior only partially in the sense that the wall body has the first and further openings. The wall body is the geometric body which is formed by the receptacle wall.

Guide Portions

The guiding portions serve to guide the containers into the receptacles during insert. Preferably, these guiding portions are formed as guiding ribs extending in a longitudinal direction of the receptacles, wherein it is preferred that in each receptable two or more of these guiding ribs are distributed at certain angular spacings from one another. Preferably, these guiding portions are not equally distributed with identical angular spacings between each other within a given receptacle.

It may be sufficient if the guiding ribs protrude radially inwards from side walls or side wall portions of the receptacles only to a relatively small extent, for example by a distance that can be of the order of one millimetre or less only. The guiding ribs or at least their front edges may be designed as planar, inclined surfaces.

The guiding ribs can extend continuously over almost the entire depth h of the receptable. Such guiding ribs are preferably inclined at an angle $\beta$ of inclination relative to longitudinal axis $L_{re\text{-}ceptacle}$ of the receptacle, wherein $\beta$ is preferably in the range from 0.01 to 1 degrees, more preferably in the range from 0.025 to 0.75 degrees, even more preferably in the range from 0.05 to 0.5 degrees and most preferably in the range 0.075 to 0.3 degrees. Generally, the angle of inclination of the guiding ribs relative to longitudinal axis $L_{receptacle}$ may be negligible in principle or it is of the order of one degree or less to facilitate the demoulding of the holding structure from an injection mould during manufacture by injection moulding.

In the alternative, the guiding ribs may also comprise separate upper portions near the upper ends of the receptacles and lower guiding portions near the lower ends of the receptacles as disclosed, for example, in US 11,286,095 B2.

Primary packaging container

A primary packaging container is the container that most closely protects the product in its distribution channels. The primary packaging container can also be referred to as retail or consumer packaging container. It follows that a test tube is not a primary packaging container.

The primary packaging containers may have any size or shape which the skilled person deems appropriate in the context of the invention. A preferred primary packaging container is a primary packaging container for a medical, pharmaceutical or cosmetical composition. Preferably, the primary packaging container is suitable for packaging parenteralia in accordance with section 3.2.1 of the European Pharmacopoeia, 7th edition from 2011. A particularly preferred primary packaging container is a vial, syringe, cartridge or ampoule.

Preferably, each of the primary packaging containers comprises, in the following sequence along its length a first end part, comprising a discharge orifice; an elongated cylindrical body region; and a further end part. Preferably, a first end part of the primary packaging container comprises a discharge orifice, which allows for discharging a medical, pharmaceutical or cosmetical composition from the primary packaging container interior of the primary packaging container. In that case, the container wall of the primary packaging container encloses the container interior only partially. A preferred primary packaging container, the first end part of which comprises a discharge orifice is a vial, a syringe or a cartridge. A preferred primary packaging container, the first end part of which does not comprise a discharge orifice is an ampoule. In that case, the container wall of the primary packaging container entirely encloses the container interior.

Additionally, or alternatively preferred, the further end part is a standing base, or comprises a further orifice, or both. In the case of a further orifice, the primary packaging container, preferably, is a syringe. In the case of a standing base, the primary packaging container, preferably, is a vial, cartridge, or ampoule. For a preferred primary packaging container, the elongated cylindrical body region follows the first end part a shoulder. This preferred primary packaging container may be a vial, syringe, cartridge, or ampoule, wherein a vial, cartridge or ampoule is preferred. Additionally, or alternatively preferred, the further end part follows the elongated cylindrical body region via a heel. In that case, the primary packaging container, preferably, is a vial, cartridge or ampoule. Preferably, the elongated cylindrical body region is a lateral region of the primary packaging container. Particularly preferable, the elongated cylindrical body region of the container wall forms a hollow cylinder. In case of a syringe, the elongated cylindrical body region is often referred to as barrel. Additionally, or alternatively preferred, the first end part comprises, more preferably consists of, from top to bottom of the primary packaging container a flange and a neck. In this case, the primary packaging container, preferably, is a vial, cartridge or ampoule.

The primary packaging container is preferably a glass container, a wall of glass (container wall) of which at least partially encloses a container interior of the primary packaging container. Preferably, the wall of glass is of a one-piece design. The wall of glass may preferably be made by blow moulding a glass melt; or by preparing a tube of a glass, preferably in form of a hollow cylinder, optionally forming the bottom of the primary packaging container from one end of the tube, thereby closing the tube at this end, and forming the top region of the primary packaging container from the opposite end of the tube. Preferably, the wall of glass is transparent. Alternatively, the container wall is preferably made from a polymer. In that case, the container wall is also preferred to be transparent.

For the use in this document, the interior volume of the primary packaging container interior represents the full volume of the interior of the container. This volume may be determined by filling the interior of the container with water up to the brim and measuring the volume of the amount of water which the interior can take up to the brim. Hence, the interior volume of the container interior as used herein is not a nominal volume as it is often referred to in the technical field of pharmacy. This nominal volume may for example be less than the interior volume by a factor of about 0.5.

Glass

The container wall of each of the primary packaging containers, preferably, comprises a glass, more preferably essentially consists of the glass. This glass may be any type of glass and may have any composition which the skilled person deems suitable in the context of the invention. Preferably, the glass is suitable for pharmaceutical packaging. Particularly preferable, the glass is of type I in accordance with the definitions of glass types in section 3.2.1 of the European Pharmacopoeia, 7th edition from 2011. Additionally, or alternatively preferable to the preceding, the glass is selected from the group consisting of a borosilicate glass, an aluminosilicate glass, and fused silica; or a combination of at least two thereof, wherein a borosilicate glass is particularly preferred. For the use in this document, a borosilicate glass is a glass which has a content of $B_2O_3$ of at least 1 wt.-%, preferably at least 2 wt.-%, more preferably at least 3 wt.-%, more preferably at least 4 wt.-%, even more preferably at least 5 wt.-%, particularly preferable in a range from 5 to 15 wt.-%, in each case based on the total weight of the glass. A preferred borosilicate glass has a content of $Al_2O_3$ of less than 7.5 wt.-%, preferably less than 6.5 wt.-%, particularly preferably in a range from 0 to 5.5 wt.-%, in each case based on the total weight of the glass. In a further aspect, the borosilicate glass has a content of $Al_2O_3$ in a range from 3 to 7.5 wt.-%, preferably in a range from 4 to 6 wt.-%, in each case based on the total weight of the glass.

Medical, Pharmaceutical and Cosmetical Compositions

In the context of the invention, every medical composition, every pharmaceutical composition and every cosmetic composition, which the skilled person deems suitable, comes into consideration. A medical composition is a composition for the use in a medical treatment. A medical composition does not necessarily comprise an active ingredient. A pharmaceutical composition is a composition comprising at least one pharmaceutically active ingredient. A preferred pharmaceutically active ingredient is a vaccine. A cosmetic composition is a composition comprising at least one cosmetically active ingredient. A preferred cosmetically active ingredient is hyaluronic acid or botulinum toxin. The medical, pharmaceutical or cosmetical composition may be fluid or solid or both, wherein a fluid composition is particularly preferred herein. A preferred solid composition is granular such as a powder, a multitude of tablets or a multitude of capsules. A further preferred medical, pharmaceutical or cosmetical composition is a parenterialium, i.e. a composition which is intended to be administered via the parenteral route, which may be any route which is not enteral. Parenteral administration can be performed by injection, e.g. using a needle (usually a hypo-dermic needle) and a syringe, or by the insertion of an indwelling catheter.

Secondary Packaging Container

The secondary packaging container may, generally, be any container which, for the skilled person, comes into consideration for accommodating the holding structure, preferably the arrangement. A preferred secondary packaging container is a tub. Particularly preferable, the secondary packaging container is a tub of a so-called nested solution as they are generally known in the technical field of transport packaging for medical, pharmaceutical and cosmetical containers. An example of a known nested solution is commercially available from Schott AG under the tradename SCHOTT iQ® platform. A preferred secondary packaging container has been prepared by deep drawing or injection moulding, where deep drawing is particularly preferred. Additionally, or alternatively preferred, the secondary packaging container is made from one or more plastics. In this context, a preferred plastic is one, selected from the group, consisting of a polycondensation polymer, preferably polyethylene terephthalate; a polyacrylate, preferably polymethylmethacrylate; and a polyolefin, preferably polypropylene or polyethylene; a polystyrene or a combination of at least two thereof.

Directions

The lateral and longitudinal directions, as referred to herein, are perpendicular to one another. The longitudinal direction, preferably, extends along the lengths of the receptacles. Preferably, any direction, which is perpendicular to the longitudinal direction comes into consideration as lateral direction. Thus, a multitude of lateral directions may derive from a single longitudinal direction. Here, the lateral directions, preferably, form a plane which is perpendicular to the longitudinal direction. Preferably, this plane is a plane of plate-like extension of the plate-shaped carrier element. Any transversal section, herein, is perpendicular to the longitudinal direction. Unless otherwise specified in the description or the particular figure:

Figure 1A   is a cross-sectional view of a primary packaging container 200 accommodated in a receptacle 101 of a holding structure 100 according to the present invention in the resting position;

Figure 1B   shows a primary packaging container 200 that can be accommodated in the receptacles 101 of the holding structure 100 according to the present invention;

Figure 2   is a cross-sectional view of a primary packaging container 200 accommodated in a receptacle 101 of a holding structure 100 according to the present invention in the lifting position;

Figure 3   is a top view of a holding structure 100 according to the present invention in which the alignment elements 106 are attached to the side walls 104 of the receptacles 101;

Figure 4   is a further top view of a holding structure 100 according to the present invention in which the alignment elements 106 are located on the top of the guide portions 109;

Figure 5   is a cross-sectional view of a receptacle 101 in which the side walls 104 of the receptacles 101 are inclined at a draft angle $\gamma$;

Figure 6   is a cross-sectional view of a receptacle 101 in which the alignment elements 106 are adapted and arranged to contact a part of the outer surface of the primary packaging containers 200 over at last a certain part of the length of the section 201' of the primary packaging container 200 that is accommodated in the receptacle 101;

Fig. 7   is the top view of the receptacle 101 shown in Fig. 6;

Figure 8A   is a cross-sectional view of a receptacle 101 in which the alignment elements 106 are provided with a concave recess 108 the curvature of which corresponds to the outer diameter of the primary packaging container 200 to be accommodated in the receptacle 101;

Figure 8B   is a top view of the alignment elements 106 in the receptacle shown in Fig. 8A;

Figure 9   is a cross-sectional view of a receptacle 101 in which the alignment elements 106 are adapted and arranged to punctually contact the outer surface of the primary packaging container 200 accommodated in the receptacle 101 at position $P_x$;

Figure 10   is a side view of a receptacle 101 in a holding structure 100 according to the present invention that is designed in such a manner that a twopart original mould with a lower mould 110 and an upper mould 111 is used for production of the holding structure 101;

Figure 11A   light microscope image of a part of the outer surface of the container wall from the Comparative Example;

Figure 11B   light microscope image of a part of the outer surface of the container wall from the Example.

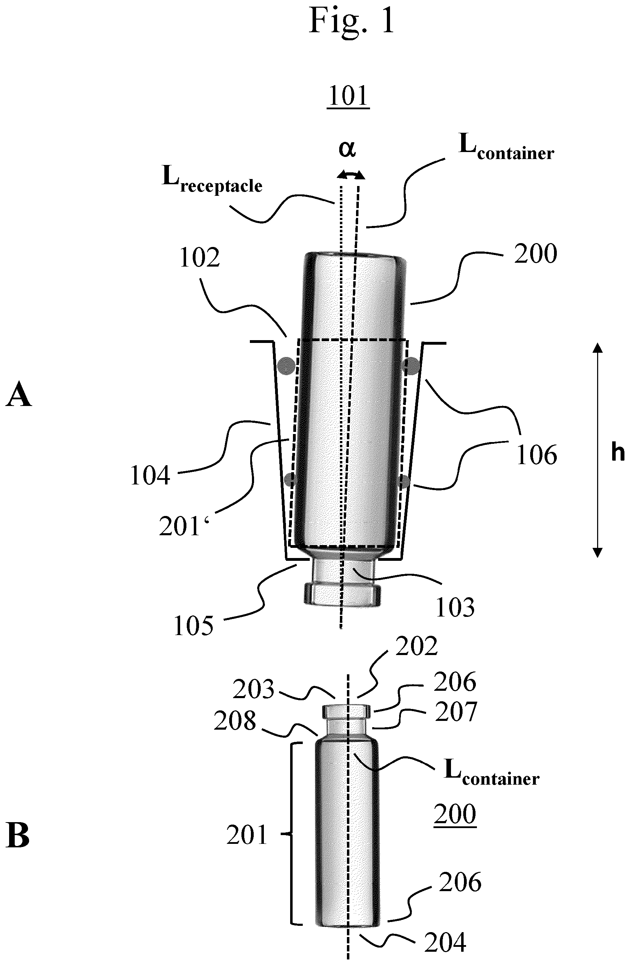

Fig. 1A is a cross-sectional view of a primary packaging container 200 accommodated in a receptacle 101 of a holding structure 100 according to the present invention in the resting position. The receptacle 101 serves to receive the primary packaging containers 200 therein at least in sections 201'. Each receptacle 101 has a depth **h,** an upper end 102 for inserting the primary packaging container 200 into the receptacle 101, a bottom end 103 having a holding portion 104 configured to limit an axial movement of the primary packaging container 200 in the receptacle 101, a circumferentially formed side wall 105 extending in a longitudinal direction from the upper end 102 to the bottom end 103 and a longitudinal axis $L_{receptable}$ that runs in a longitudinal direction through the centre of the receptacle 101. In Fig. 1A the section 201' of the elongated cylindrical body region 201 of the primary packaging container 200 that in the resting position is accommodated in the receptacle is marked by a dashed parallelogram. As shown in Fig. 1A, the holding structure according to the present invention each receptacle 101 comprises alignment elements 106 (in Fig. 1A shown in the form of grey circles) that project radially into the receptacle 101 and that are adapted and arranged to contact a part of the outer surface of the elongated cylindrical body region 201 of a primary packaging container 200 accommodated in the receptacle 101. The alignment elements 106 in each receptacle 101 are adapted and arranged to contact a part of the outer surface of the elongated cylindrical body region 201 in such a way that a primary packaging container 200 accommodated in the receptacle 101 in the resting position is tilt by an angel $\alpha$ of not more than 2.4 degrees, wherein the tilting angle $\alpha$ is the angle that is formed between longitudinal axis $L_{receptacle}$ and longitudinal axis $L_{container}$ of the primary packaging container 200 accommodated in the receptacle 101. The resting position is the position in which the holding portion 104 limits the axial movement of the primary packaging container 200 accommodated in the in the receptacle 101. The resting position is thus the position in which the primary packaging container 200 is accommodated up to the maximum depth in the receptacle 101. As can be seen, in the receptacle 101 shown in Fig. 1A in the resting position the holding portions come into contact with the shoulder 208 of the primary packaging container 200.

Fig. 1B shows a primary packaging container 200 in the form of a vial that can be accommodated in the receptacles 101 of the holding structure 100 according to the present invention. The primary packaging container 200 may comprise a container wall which partially surrounds a container interior. The container wall forms, in the following sequence from top to bottom, a first end part 202, comprising a discharge orifice 203, an elongated cylindrical body region 201, and a further end part 204. The elongated cylindrical body region 201 is a preferably hollow cylinder. The further end part 204 comprises a standing base 205. Besides the discharge orifice 203, the first end part 202 consists of a flange 206 and a neck 207. The elongated cylindrical body region 201 follows the first end part 202 via a shoulder 208. The further end part 204 follows the elongated cylindrical body region 201 via a heel 209. The container wall is made from type I borosilicate glass.

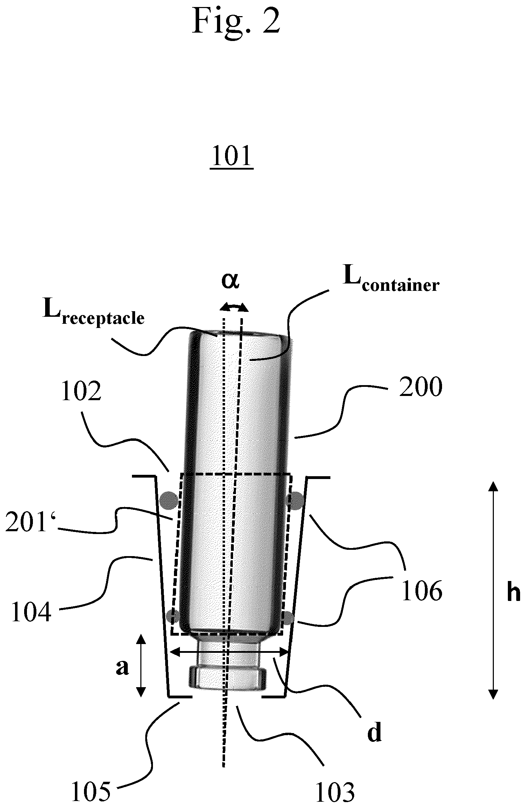

Fig. 2 is a cross-sectional view of a primary packaging container 200 accommodated in a receptacle 101 of a holding structure 100 according to the present invention in the lifting position. The lifting position is the position in which the primary packaging container 200 is lifted out of the resting position in a direction away from the bottom end 103 to a lifting height **a,** wherein a is 10 to 50 %, preferably 15 to 45 % and more preferably 20 to 40 % of the depth h of the receptacle. Such a lifting of primary packaging containers in a holding structure occurs, for example, if in an automated filling process optical sensors are used to confirm the correctness of the filling height. As can be seen in Fig. 2, the alignment elements 106 preferably also ensure that even in the lifting position the tilting angle α' does not exceed a certain maximum value.

Fig. 3 is a top view of a holding structure 100 according to the present invention in which the alignment elements 106 are attached to the side walls 104 of the receptacles 101. As can be seen, the receptacles 101 are arranged in a regular arrangement. An upper side of the holding structure 100 is formed as a plate-shaped support 107 and the receptacles 101 project vertically from the plate-shaped support 107. The side wall 104 of each receptacle 101 is formed as a common partition between each two directly adjacent receptacles 101 of the plurality of receptacles 101. As can also be seen in Fig. 3, holding protrusions 105 acting as holding portions 105 are provided at the bottom ends 103 of the receptacles 101, which extend radially inwards into the receptacles 101. Each receptacle 101 may, for example, have two holding protrusions 105 which are diametrically opposite to each other. In the holding structure 100 shown in Fig. 3 the alignment elements 106 are provided as point-engaging contact elements 106 in the upper area of the receptacle 101.

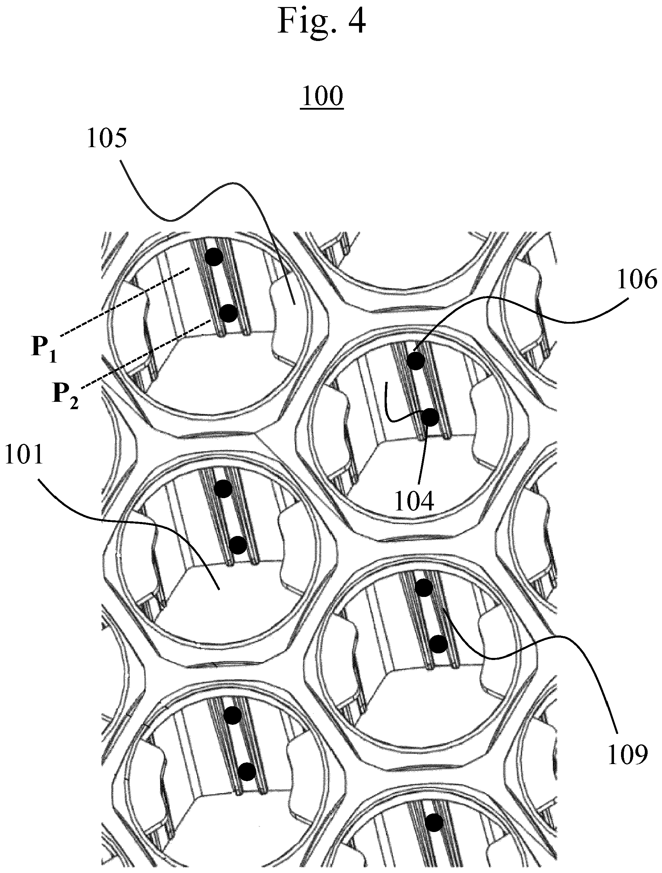

Fig. 4 is a further top view of a holding structure 100 according to the present invention in which the alignment elements 106 are provided as point-engaging contact elements 106 that are located on the top of the guide portions 109 in the receptacles 101. The guide portions 109 are adapted and arranged to guide the primary packaging containers 200 as they are inserted into the receptacles 101. In the holding structure 100 shown in Fig. 4 the guide portions 109 are formed as guiding ribs 109 extending in a longitudinal direction of the receptacles 101.

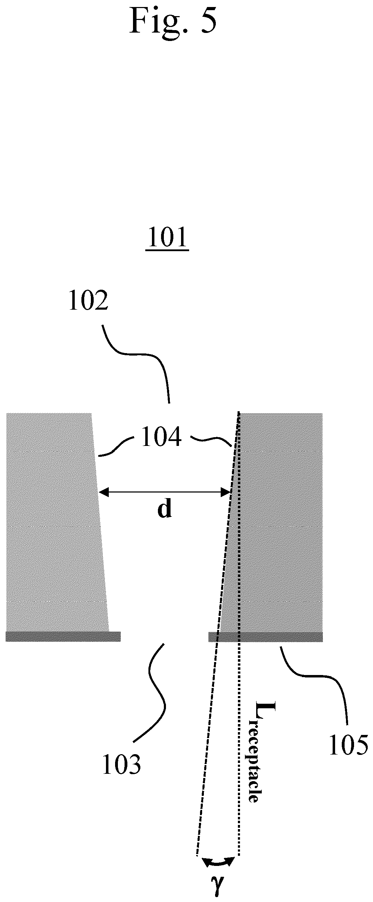

Fig. 5 is a cross-sectional view of a receptacle 101 in which the side walls 104 of the receptacles 101 are inclined at a draft angle γ so that the inner diameter **d** of the receptacle 101 constantly increases from the bottom end 103 to the upper end 102. In this context the draft angle γ is preferably in the range from 0.01 to 1 degree, more preferably in the range from 0.025 to 0.75 degrees, even more preferably in the range from 0.05 to 0.5 degrees and most preferably in the range 0.075 to 0.3 degrees.

Fig. 6 is a cross-sectional view of a receptacle 101 in which the alignment elements 106 are adapted and arranged to contact a part of the outer surface of the primary packaging containers 200 over at last a certain part of the length of the elongated cylindrical body region 201 that is accommodated in the receptacle 101. In the embodiment shown in Fig. 6, these longitudinal alignment elements 106 are located on the top of the guiding ribs 109 and extend over almost the entire length of these guiding ribs 106.

Fig. 7 is the top view of a part of a holding structure 100 according to the present invention as shown in Fig. 6 in which alignment elements 106 that contact the outer surface of the primary packaging containers 200 accommodated in the receptacle 101 are located on top of the surface of guiding ribs 109 like the arrangement in Fig. 6. In the receptacle 101 shown in Figs. 6 and 7, the inner diameter of the receptacle 101 as defined by the dimensions of the alignment elements 106 as determined in a position $P_2$ in a height of 13 mm above the bottom end 103 of the receptacle 101 is 15.95 mm, whereas the inner diameter of the receptacle 101 as defined by the dimensions of the alignment elements 106 determined at a position $P_1$ in a distance of 33.25 mm from position $P_2$ is 16.11 mm.

Fig. 8A is a cross-sectional view of a receptacle 101 in which the alignment elements 106 are provided with a concave recess 108 the curvature of which corresponds to the outer diameter of the primary packaging container 200 to be accommodated in the receptacle 101. In the embodiment shown in Fig. 8A, the receptacle 101 comprises such alignment elements only in the upper area close to the upper end 102 of the receptacle, wherein - as shown in Fig. 8 B - two such alignment elements 106 may be arranged opposite each other in such a way that their concave recesses 108 together form a circular recess in which the elongated cylindrical body region 201 of the primary packaging container 200 is enclosed.

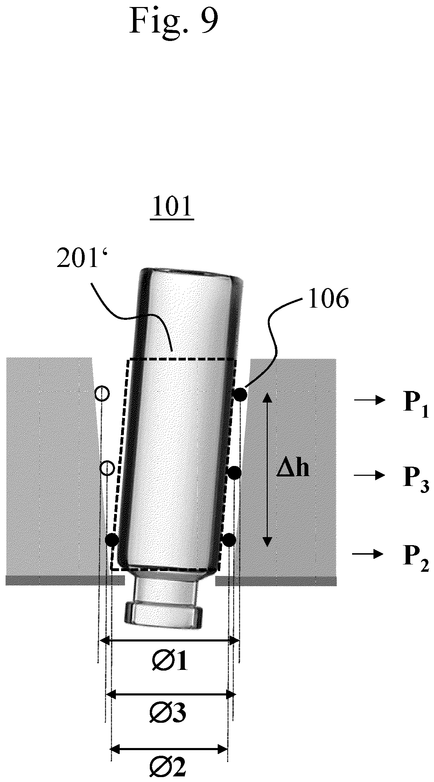

Fig. 9 is a cross-sectional view of a receptacle 101 in which the alignment elements 106 are adapted and arranged to punctually contact the outer surface of the primary packaging container 200 accommodated in the receptacle 101 at position $P_x$. In the embodiment shown in Fig. 8, these point-engaging contact elements 106 may be localized in three different heights within a given receptacle, indicated as positions $P_1$ near the upper end 102 of the receptacle 101, $P_2$ near the bottom end 103 of the receptacle 101 and $P_3$ located between $P_1$ and $P_2$. The alignment elements at position $P_1$ are preferably located along a first circle located in a first plane and the alignment elements at position $P_2$ are located along a second circle located in a second plane, the first and the second plane being parallel to each other and being perpendicular to longitudinal axis $L_{receptable}$, wherein the diameter $\varnothing_1$ of the first circle at position $P_1$ is larger than the diameter $\varnothing_2$ of the second circle at position $P_2$. In case of a receptacle 101 in which the alignment elements 106 are located at three different positions $P_1$, $P_2$ and $P_3$ as shown in Fig. 8, the alignment elements 101 at position $P_3$ are preferably also located along a third circle having a diameter $\varnothing_3$, wherein $\varnothing_1 > \varnothing_3 > \varnothing_2$.

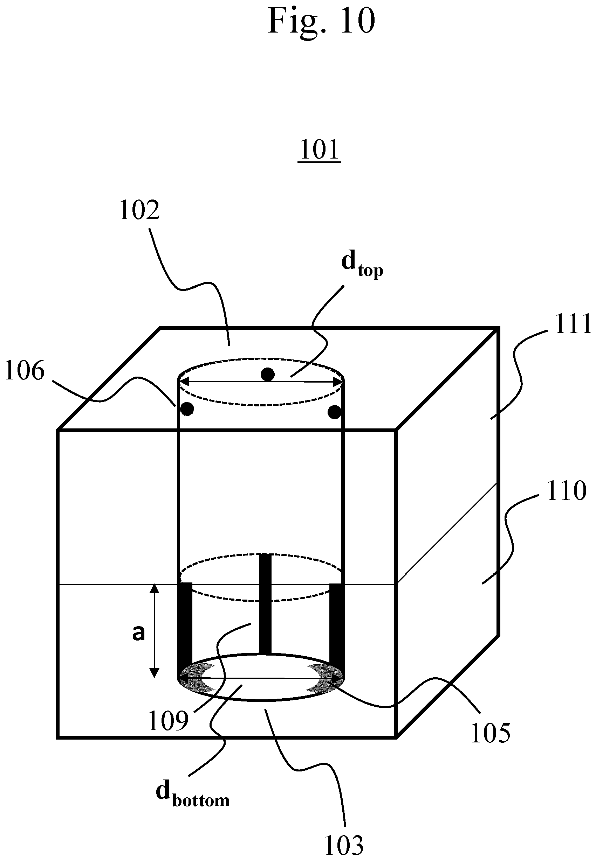

Fig. 10 is a side view of a receptacle 101 in a holding structure 100 according to the present invention that is designed in such a manner that a two-part original mould with a lower mould 110 and an upper mould 111 is used for production of the holding structure 101, wherein the alignment elements 106 are located in part of the receptacle formed by the upper mould 111. Using such a two-component mould allows the provision of receptacles 101 the side walls of which are not inclined at all so that the inner diameter $d_{bottom}$ of the receptacle at the bottom end corresponds to the inner diameter $d_{top}$ at the upper end. Such a receptacle allows the accommodation of primary packaging containers with a particularly low tilting angel $\alpha$ even if the containers are lifted in a lifting height **a.**

TEST METHODS

For the determination of the abrasion of the outer surface of the primary packaging containers in the form of a cartridge inserted into the receptacle of a holding structure the cartridge was first tempered for 24 hours at 250°C in an oven. After the cartridges had cooled down to around 24°C, they were manually inserted into a nest with receptacles comprising guiding ribs and removed by actively rubbing the cartridges along these guiding ribs. The outer surface of the cartridges was then visually inspected using light microscopy.

Example:

In a holding structure as shown in Fig. 6 comprising receptacles with a draft angle $\gamma$ of 0.2 degrees the guiding ribs were modified by attaching thereto within each receptacle alignment elements such that the inner diameter defined by these alignment elements is 15.95 mm at a position $P_2$ 13 mm above the bottom end of the receptacle and 16.11 mm at a position $P_1$. The vertical distance between heights $P_1$ and $P_2$ is 33.25 mm and the tilting angle $\alpha$ is 1.16 degrees.

Comparative Example

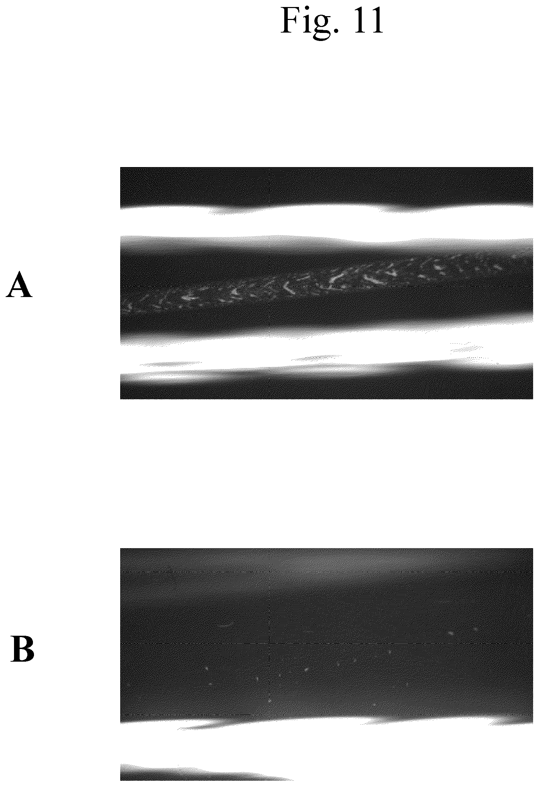

The same holding structure was used as in the Example, with the sole difference that the guiding ribs were not modified with any alignment elements.
As can be seen in Figs. 11A and 11B, the alignment elements help to significantly reduce the abrasion of the outer surface of the cartridges when they are moved inside the receptacles.

**LIST OF REFERENCE NUMBERS**

| | |
|---|---|
| **100** | holding structure |
| **101** | receptacle |
| **102** | upper end of receptacle 101 |
| **103** | bottom end of receptacle 101 |
| **104** | side wall of receptacle 101 |
| **105** | holding portion of receptacle 101 |
| **106** | alignment elements |
| **107** | plate-shaped carrier element |
| **108** | concave recess |
| **109** | guide portions |
| **110** | lower mould |
| **111** | upper mould |
| **200** | primary packaging container |
| **201** | elongated cylindrical body region of the primary packaging container 200 |
| **201'** | section of the elongated cylindrical body region 201 of the primary packaging container 200 that is accommodated in the receptacle 101 |
| **202** | first end part of the primary packaging container 200 |
| **203** | discharge orifice |
| **204** | further end part of the primary packaging container 200 |
| **205** | standing base |
| **206** | flange |
| **207** | neck |
| **208** | shoulder |
| **300** | transport unit according to the invention |
| **301** | arrangement according to the invention |
| **302** | secondary packaging container |
| **303** | lid |

**Claims**

**1.** A holding structure (100) for concurrently holding a plurality of primary packaging containers (200) for substances for pharmaceutical, medical or cosmetic applications, each primary packaging container (200) comprising an elongated cylindrical body region (201) having a longitudinal axis $L_{container}$ that runs in a longitudinal direction through the centre of the elongated cylindrical body region (201), the holding structure (100) comprising:

- a plurality of receptacles (101) for accommodating at least a section (201') of the elongated cylindrical body region (201) of the primary packaging container (200), each receptacle (101) having a depth **h,** an upper end (102) for inserting the primary packaging container (200) into the receptacle (101), a bottom end (103) having a holding portion (104) configured to limit an axial movement of the primary packaging container (200) in the receptacle (101), a circumferentially formed side wall (105) extending in a longitudinal direction from the upper end (102) to the bottom end (103) and a longitudinal axis $L_{receptable}$ that runs in a longitudinal direction through the centre of the receptacle (101);
- alignment elements (106) in each receptacle (101) that project radially into the receptacle (101) and that are adapted and arranged to contact a part of the outer surface of the elongated cylindrical body region (201) of a primary packaging container (200) accommodated in the receptacle (101);

wherein the alignment elements (106) in each receptacle (101) are adapted and arranged to contact a part of the outer surface of the elongated cylindrical body region (201) in such a way that a primary packaging container (200) accommodated in the receptacle (101) in a resting position is tilt by an angel $\alpha$ of not more than 2.4 degrees, the tilting angle $\alpha$ being the angle that is formed between longitudinal axis $L_{receptacle}$ and longitudinal axis $L_{container}$ of the primary packaging container (200) accommodated in the receptacle (101) and the resting position being the position in which the holding portion (104) limits the axial movement of the primary packaging container (200) accommodated in the in the receptacle (101).

2. The holding structure (100) according to claim 1, wherein the alignment elements (106) in each receptacle (101) are adapted and arranged to contact a part of the outer surface of the elongated cylindrical body region (201) in such a way that a primary packaging container (200) accommodated in the receptacle (101) in a lifting position is still tilt by an angel $\alpha'$ of not more than 2.4 degrees, the tilting angle $\alpha'$ being the angle that is formed between longitudinal axis $L_{receptacle}$ and longitudinal axis $L_{container}$ of the primary packaging container (200) accommodated in the receptacle and the lifting position being the position in which the primary packaging container (200) is lifted out of the resting position in a direction away from the bottom end (103) to a lifting height **a,** wherein a is 10 to 50 % of the depth **h** of the receptacle.

3. The holding structure (100) according to claim 1 or 2, wherein the holding structure (100) is formed by injection moulding from a plastic material and wherein the side walls (105) of the receptacles (101) are inclined at a draft angle $\gamma$ in relation to longitudinal axis $L_{receptable}$ so that the inner diameter **d** of the receptacle (101) constantly increases from the bottom end (103) to the upper end (102).

4. The holding structure (100) according to any of claims 1 to 3, wherein the holding structure (100) is formed by injection moulding from a plastic material, wherein the holding structure (100) is designed in such a manner that a two-part original mould with a lower mould and an upper mould is used for production by injection moulding from the plastic material such that the side walls (105) of the receptacles (101) are not inclined at a draft angle $\gamma$ in relation to longitudinal axis $L_{receptable}$ so that the inner diameter $d_{bottom}$ of the receptacle (101) at the bottom end (103) corresponds to the inner diameter $d_{top}$ at the upper end (102).

5. The holding structure (100) according to any of claims 1 to 4, wherein

- the receptacles (101) are arranged in a regular arrangement,
- an upper side of the holding structure (100) is formed as a plate-shaped carrier element (107), and
- the receptacles (101) project vertically from the plate-shaped carrier element (107);

wherein the side wall (104) is formed as a common partition between each two directly adjacent receptacles (101) of the plurality of receptacles (101).

6. The holding structure (100) according to any of claims 1 to 5, wherein in each receptacle (101) the alignment elements (106) comprise a plurality of longitudinal alignment elements (106) running in a longitudinal direction from the upper end (102) to the bottom end (103) of the receptacle (101) and extending over at least 50 % of the entire depth **h** of the receptable (101), wherein the alignment elements (106) are adapted and arranged to contact a part of the outer surface of the primary packaging container (200) over at last a certain part of the length of the elongated cylindrical body region (201) that is accommodated in the receptacle (101).

7. The holding structure (100) according to any of claims 1 to 5, wherein in each receptacle (101) there are one more positions $P_x$ at which at least two alignment elements (106) are provided with a concave recess (108) the curvature of which corresponds to the outer diameter of the primary packaging container (200) to be accommodated in the receptacle (101), wherein the alignment elements (106) are adapted and arranged to contact the outer surface of the primary packaging container (200) over at least a part of the circumference of the elongated cylindrical body region (201') that is accommodated in the receptacle (101).

8. The holding structure (100) according to any of claims 1 to 5, wherein in each receptacle (101) there are one more positions $P_x$ at which at least two alignment elements (106) are provided, wherein the alignment elements (106) are adapted and arranged to punctually contact the outer surface of the primary packaging container (200) on at least a part of the elongated cylindrical body region (201) that is accommodated in the receptacle (101).

9. The holding structure (100) according to any of claims 1 to 8, wherein the alignment elements (106) are located on the top of the side walls (104).

10. The holding structure (100) according to any of claims 1 to 9, wherein the holding structure (100) further comprises

    - guide portions (109) adapted and arranged to guide the primary packaging containers (200) as they are inserted into the receptacles (101), the guide portions (109) being formed as guiding ribs (109) extending in a longitudinal direction of the receptacles (101);

    wherein the alignment elements (106) are located on the top of the guide portions (109).

11. An arrangement comprising:

    - the holding structure (100) according to any of the claims 1 to 10,
    - a plurality of primary packaging containers (200), each primary packaging container (200) being accommodated in one of the receptacles (101) of the holding structure (100).

12. A transport unit comprising:

    - the arrangement according to claim 11, and
    - a secondary packaging container,

wherein the holding structure (100) and the plurality of primary packaging containers (200) are arranged completely in the secondary packaging container.

13. A process for filling primary packaging containers with a pharmaceutical, medical, or cosmetic composition, the process comprising the steps:

    A) providing an arrangement according to claim 11;
    B) filling each a primary packaging containers accommodated in the receptacles of the holding structure with the liquid pharmaceutical composition in an automated filling machine.

14. The process according to claim 13, wherein in the automated filling machine a needle is positioned above the opening of the primary packaging container, followed by an injection of the pharmaceutical, medical, or cosmetic composition through the needle into the container, wherein the primary packaging container is filled with the pharmaceutical, medical, or cosmetic composition following the positioning of the needle above the corresponding primary packaging container.

15. A use of the holding structure (100) according to any of the claims 1 to 10, or of the arrangement according to claim 11, or of the transport unit according to claim 12 in each case in a filling process according to claims 13 and 14.

# Fig. 1

<u>101</u>

A

B

# Fig. 2

<u>101</u>

# Fig. 3

<u>100</u>

# Fig. 4

## 100

# Fig. 5

<u>101</u>

# Fig. 6

## 101

# Fig. 7

## 101

# Fig. 8

# Fig. 9

# Fig. 10

# Fig. 11

A

B

## EUROPEAN SEARCH REPORT

**Application Number**

EP 24 17 0195

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/298610 A1 (KOMANN CHRISTIAN [CH] ET AL) 3 October 2019 (2019-10-03) * figures 1, 2, 3, 4A-B, 5A-B, 6, 7, 8A-C * * paragraphs [0068] - [0087] * | 1-15 | INV. A61M5/00 A61J1/16 |
| X | US 2019/343721 A1 (KOMANN CHRISTIAN [CH] ET AL) 14 November 2019 (2019-11-14) * figures 1A-F, 2A-D, 3A-G, 4A-C * * paragraphs [0069] - [0107] * | 1-15 | |
| X | US 2018/208377 A1 (KLOKE ARNE [CH] ET AL) 26 July 2018 (2018-07-26) * figures 1a-h, 2a-e, 3a-e, 4a-f, 5a-e, 6a-d, 7a-h, 8a-c, 9 * * paragraphs [0105] - [0162] * | 1-14 | |
| X | US 2020/156824 A1 (KOMANN CHRISTIAN [CH] ET AL) 21 May 2020 (2020-05-21) * figures 1A-F, 2A-C * * paragraphs [0028] - [0078] * | 1-15 | TECHNICAL FIELDS SEARCHED (IPC) |
| A | EP 3 381 828 A1 (SCHOTT KAISHA PVT LTD [IN]) 3 October 2018 (2018-10-03) * the whole document * | 1-15 | A61M A61J |

The present search report has been drawn up for all claims

| Place of search | Date of completion of the search | Examiner |
|---|---|---|
| Munich | 24 September 2024 | Benes, Václav |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 0195

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2024

| Patent document cited in search report | | | Publication date | Patent family member(s) | | | Publication date |
|---|---|---|---|---|---|---|---|
| US 2019298610 | A1 | | 03-10-2019 | CN | 110099706 | A | 06-08-2019 |
| | | | | DE | 202016107209 | U1 | 22-03-2018 |
| | | | | EP | 3534986 | A1 | 11-09-2019 |
| | | | | US | 2019298610 | A1 | 03-10-2019 |
| | | | | WO | 2018114103 | A1 | 28-06-2018 |
| US 2019343721 | A1 | | 14-11-2019 | CA | 3042905 | A1 | 14-11-2019 |
| | | | | CN | 110481984 | A | 22-11-2019 |
| | | | | CN | 115384923 | A | 25-11-2022 |
| | | | | DE | 102018111491 | A1 | 14-11-2019 |
| | | | | EP | 3569314 | A1 | 20-11-2019 |
| | | | | EP | 3862095 | A1 | 11-08-2021 |
| | | | | JP | 7424758 | B2 | 30-01-2024 |
| | | | | JP | 2019198650 | A | 21-11-2019 |
| | | | | KR | 20190130481 | A | 22-11-2019 |
| | | | | US | 2019343721 | A1 | 14-11-2019 |
| US 2018208377 | A1 | | 26-07-2018 | CN | 108341142 | A | 31-07-2018 |
| | | | | DE | 102017101398 | A1 | 26-07-2018 |
| | | | | EP | 3354589 | A1 | 01-08-2018 |
| | | | | US | 2018208377 | A1 | 26-07-2018 |
| US 2020156824 | A1 | | 21-05-2020 | CN | 111196415 | A | 26-05-2020 |
| | | | | DE | 202018106510 | U1 | 20-02-2020 |
| | | | | EP | 3653526 | A1 | 20-05-2020 |
| | | | | US | 2020156824 | A1 | 21-05-2020 |
| EP 3381828 | A1 | | 03-10-2018 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11464702 B2

- US 11286095 B2

**Non-patent literature cited in the description**

- European Pharmacopoeia. 2011